# EUROPEAN PATENT APPLICATION

(11) **EP 4 726 732 A1**
(43) Date of publication of application: **15.04.2026**
(21) Application number: 25207157.6
(22) Date of filing: 07.10.2025
(51) Int. Cl.: G16H 20/40, A61C 13/00, G16H 30/40, A61B 5/00, A61B 6/03, A61C 8/00, A61C 9/00, G05B 19/4099, G06F 21/60, G06N 3/045, G06N 3/08, G16H 40/63, G16H 50/20, G16H 50/70

(54) **PROSTHESIS GENERATING METHOD USING THREE DIMENSIONAL SCAN DATA AND COMPUTER READABLE MEDIUM HAVING PROGRAM FOR PERFORMING THE METHOD**

(30) Priority: 11.10.2024 KR 20240138828; 14.11.2024 WO PCT/KR2024/017927
(71) Applicant: Imagoworks Inc., Seoul 06034 (KR)
(72) Inventor: JUNG, Daeil, 14687 Bucheon-si Gyeonggi-do (KR)
(74) Representative: BCKIP Part mbB

(57) **Abstract**

A prosthesis generating method using three dimensional scan data includes receiving encryption oral data, receiving a model information of an artificial intelligence neural network, determining meta data of the encryption oral data, determining an operation predicted value based on the model information and the meta data and determining a size of a sub-operator based on the operation predicted value. The size of the sub-operator is controlled based on the operation predicted value.

## Description

This application claims priority to Korean Patent Application No. 10-2024-0138828, filed on October 11, 2024 in the Korean Intellectual Property Office KIPO and International Patent Application No. PCT/KR2024/017927 filed on November 14, 2024.

### BACKGROUND

### 1. Field

Embodiments of the present disclosure relate to a prosthesis generating method using three dimensional scan data and computer readable medium having program for performing the method. More particularly embodiments of the present disclosure relate to a prosthesis generating method using three dimensional scan data, a cloud environment and an artificial intelligence neural network and computer readable medium having program for performing the method.

### 2. Description of the Related Art

With an advancement of artificial intelligence (AI) technology, a method of generating a prosthesis is gradually shifting to a method using AI. However, when generating a prosthesis using AI, production time may be delayed depending on user's computing environment.

### SUMMARY

Embodiments of the present disclosure provide a prosthesis generating method using three dimensional scan data.

According to embodiments, a prosthesis generating method using three dimensional scan data includes receiving encryption oral data, receiving a model information of an artificial intelligence neural network, determining meta data of the encryption oral data, determining an operation predicted value based on the model information and the meta data and determining a size of a sub-operator based on the operation predicted value. The size of the sub-operator is controlled based on the operation predicted value.

In an embodiment, the encryption oral data may be generated based on oral data. The artificial intelligence neural network may be selected based on a feature of the oral data.

In an embodiment, when the sub-operator is in an inactivation state, the sub-operator may be generated based on the size. The generated sub-operator may receive the artificial intelligence neural network corresponding to the model information. The generated sub-operator may generate prosthesis data based on the received artificial intelligence neural network and the encryption oral data.

In an embodiment, when the sub-operator is in a waiting state, the sub-operator may receive the artificial intelligence neural network corresponding to the model information. The generated sub-operator may generate prosthesis data based on the received artificial intelligence neural network and the encryption oral data.

In an embodiment, when the sub-operator generates the prosthesis data, a state of the sub-operator may be changed to the waiting state.

In an embodiment, the meta data may include an information of a tooth number and a tooth count of oral data corresponding to the encryption oral data. When the tooth count is greater than a reference tooth count, the size of the sub-operator may be increased.

In an embodiment, the meta data may further include prosthesis type data.

In an embodiment, the method may further include receiving a generation request number. When the generation request number is greater than a reference generation request number, a size of an activation sub-operation block which includes the sub-operator which is in an activation state may be increased.

In an embodiment, the sub-operator may receive the artificial intelligence neural network from a data storage. The data storage may include a plurality of artificial intelligence neural networks. The data storage may apply the artificial intelligence neural network corresponding to the model information to the sub-operator.

In an embodiment, the sub-operator may generate prosthesis data based on the received artificial intelligence neural network and the encryption oral data.

In an embodiment, the method may further include receiving a generation request number. When the generation request number is greater than a reference generation request number, a size of an activation sub-operation block which includes the sub-operator which is in an activation state may be increased.

In an embodiment, the method may further include receiving a generation request number. When the generation request number is greater than a reference generation request number, a number of the sub-operator which is in an activation state may be increased.

In an embodiment, the model information may be determined based on oral data corresponding to the encryption oral data. The model information may be determined based on a tooth number of the oral data and a prosthesis type.

In an embodiment, a method of generating the encryption oral data may include generating an encryption key of oral data and encrypting the oral data based on the encryption key. When the encryption key and the encryption oral data are same, the encryption oral data may be received.

In an embodiment, a program for executing the method for prosthesis generating method using three dimensional scan data may be stored in a computer readable medium.

As described above, according to the prosthesis generating method using three dimensional scan data and computer readable medium having program for performing the method, the prosthesis data may be generated using the selected artificial intelligence neural network. Accordingly, the prosthesis data suitable for user's oral data may be generated. Additionally, a manufacturing speed of prosthesis may be improved.

Additionally, the size of the data operator in which the prosthetic data is generated may be changed based on the model information of the artificial intelligence neural network and the meta data of the oral data. Accordingly, the operating speed of the prosthesis data may be improved.

Additionally, the data operator in which the prosthetic data is generated may be a cloud server environment. Accordingly, the operating speed of the prosthesis data may be further improved.

### BRIEF DESCRIPTION OF THE DRAWINGS

FIG. 1 is a flowchart illustrating a method for generating a prosthesis.
FIG. 2 is a block diagram illustrating an example of a prosthesis generating system performing the method for generating a prosthesis of FIG. 1.
FIG. 3 is a flowchart illustrating a method for encrypting an oral data of FIG. 1.
FIG. 4 is a block diagram illustrating an example of an operation of a prosthesis generating system which performs the method for encrypting an oral data of FIG. 3.
FIG. 5 is a block diagram illustrating an example of an operation of a prosthesis generating system which performs the method for encrypting an oral data of FIG. 3.
FIG. 6 is a flowchart illustrating a method for selecting an artificial intelligence neural network corresponding to the oral data of FIG. 1.
FIG. 7 is a diagram illustrating a feature of the oral data of FIG. 6.
FIG. 8 is a block diagram illustrating an example of an operation of a prosthesis generating system which performs the method for selecting an artificial intelligence neural network corresponding to the oral data of FIG. 6.
FIG. 9 is a flowchart illustrating a method for determining a sub-operator in which a selected artificial intelligence neural network is operated.
FIG. 10 is a block diagram illustrating an example of an operation of a data operator included in the prosthesis generating system of FIG. 1 which performs the method for determining a sub-operator in which a selected artificial intelligence neural network is operated.
FIG. 11 is a flowchart illustrating an example of an operation of a data operator of FIG. 10.

### DETAILED DESCRIPTION OF THE EMBODIMENTS

The specific structural or functional descriptions of embodiments of the present inventive concept disclosed herein are merely illustrated for the purpose of explaining the embodiments of the present inventive concept. The embodiments of the present inventive concept may be implemented in various forms and should not be construed as being limited to the embodiments described herein.

The present inventive concept is capable of various modifications and having various forms. Specific embodiments are illustrated in the drawings and described in detail in the text. However, this is not intended to limit the present inventive concept to the specific disclosed forms, and it should be understood to include all modifications, equivalents, and substitutes that fall within the spirit and technical scope of the present inventive concept.

Terms such as first and second may be used to describe various components, but the components should not be limited by these terms. These terms may be used merely to distinguish one component from another component. For example, a first component may be referred to as a second component without departing from the scope of the present inventive concept, and likewise, the second component may also be referred to as the first component.

When a component is referred to as being "connected" or "coupled" to another component, it may be directly connected or coupled to the other component, or there may be another component in between. On the other hand, when a component is referred to as being "directly connected" or "directly coupled" to another component, it should be understood that there is no other component in between. Other expressions used to describe the relationships between components, such as "between" and "immediately between" or "adjacent to" and "directly adjacent to," should be interpreted in the same manner.

The terminology used in this application is merely for the purpose of describing particular embodiments and is not intended to limit the present inventive concept. Unless explicitly stated otherwise, the singular expressions include the plural expressions as well. In this application, the terms such as "include" or "have" are intended to designate that the stated features, numbers, steps, operations, components, parts, or combinations thereof exist, and are not intended to preclude the possibility that one or more other features, numbers, steps, operations, components, parts, or combinations thereof may also exist or be added.

Unless otherwise defined, all terms used herein including technical or scientific terms have the same meaning as commonly understood by one of ordinary skill in the art to which this inventive concept belongs. Terms defined in commonly used dictionaries are to be interpreted as having meanings consistent with their use in the relevant technical field and should not be interpreted in an idealized or overly formal sense unless expressly so defined in this application.

Meanwhile, in cases where an embodiment may be implemented otherwise, functions or operations specified in a specific block may occur in an order different from that specified in the flowchart. For example, two successive blocks may in fact be executed substantially simultaneously, or the blocks may sometimes be executed in reverse order depending on the functions or operations involved.

Hereinafter, preferred embodiments of the present inventive concept will be described in more detail with reference to the accompanying drawings. In the drawings, the same reference numerals denote the same components, and repeated descriptions of the same components will be omitted.

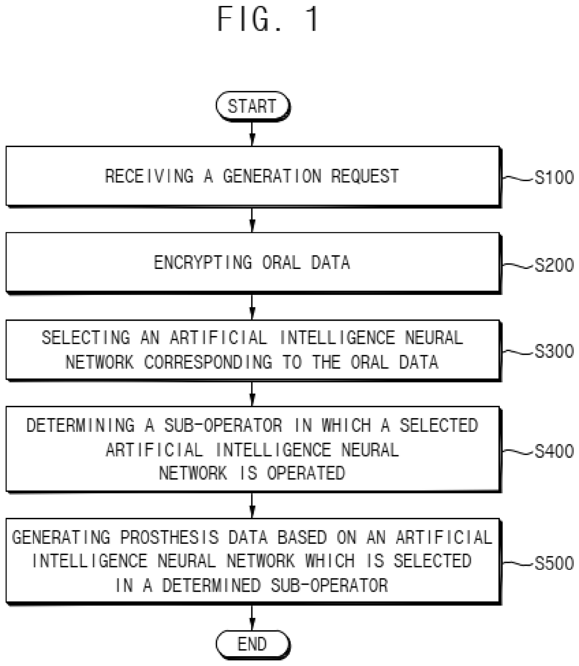

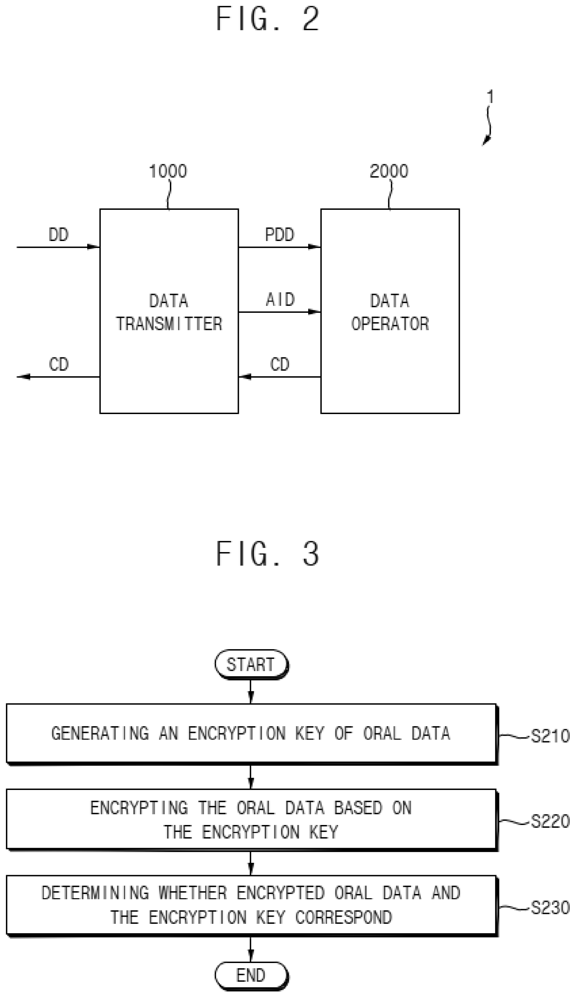

FIG. 1 is a flowchart illustrating a method for generating a prosthesis. FIG. 2 is a block diagram illustrating an example of a prosthesis generating system 1 performing the method for generating a prosthesis of FIG. 1.

Referring to FIG. 1 and FIG. 2, a method for generating a prosthesis may include receiving a generation request (S100), encrypting oral data DD (S200), selecting an artificial intelligence neural network corresponding to the oral data DD (S300), determining a sub-operator in which a selected artificial intelligence neural network is operated (S400) and generating prosthesis data CD based on an artificial intelligence neural network which is selected in a determined sub-operator (S500).

In the present embodiment, the method for generating the prosthesis may be performed by a computing device.

The prosthesis generating system 1 may include a data transmitter 1000 and a data operator 2000.

The data transmitter 1000 may receive oral data DD. The data transmitter 1000 may receive prosthesis data CD from the data operator 2000. The data transmitter 1000 may output the prosthesis data CD. The data transmitter 1000 may output a prosthesis generation request to the data operator 2000. The data transmitter 1000 may select an artificial intelligence neural network corresponding to the oral data DD. The data transmitter 1000 may output an model information AID of the artificial intelligence neural network corresponding to the oral data DD to the data operator 2000.

The data transmitter 1000 may request an encryption key corresponding to the oral data DD from the data operator 2000. The data operator 2000 may output the encryption key to the data transmitter 1000. The data transmitter 1000 may encrypt the oral data DD based on the encryption key. The oral data DD which is encrypted may be encrypted oral data PDD. The data transmitter 1000 may output the encrypted oral data PDD to the data operator 2000. The oral data DD is image data including the oral cavity of a patient. For example, the oral data DD may be three dimensional scan data generated by scanning the oral cavity of a patient with an oral scanner. For example, the oral data may be computed tomography (CT) data corresponding to the oral cavity of a patient. For example, the oral data may be magnetic resonance imaging (MRI) data corresponding to the oral cavity of a patient.

The data transmitter 1000 may perform receiving a generation request (S100), encrypting oral data DD (S200) and selecting an artificial intelligence neural network corresponding to the oral data DD (S300). In an embodiment, the data transmitter 1000 may be a computing environment of user.

The data operator 2000 may receive encrypted oral data PDD and model information AID. The data operator 2000 may generate the prosthesis data CD based on the encrypted oral data PDD using an artificial intelligence neural network corresponding to the model information AID. The data operator 2000 may store a plurality of artificial intelligence neural networks. The data operator 2000 may determine an artificial intelligence neural network corresponding to the model information AID.

For example, the data operator 2000 may select an artificial intelligence neural network corresponding to the model information AID. The data operator 2000 may output prosthesis data CD to the data transmitter 1000.

The data operator 2000 may perform determining a sub-operator in which a selected artificial intelligence neural network is operated (S400) and generating prosthesis data CD based on an artificial intelligence neural network which is selected in a determined sub-operator (S500). In an embodiment, the data operator 2000 may be a data processing server. In an embodiment, the data operator 2000 may be a cloud server environment.

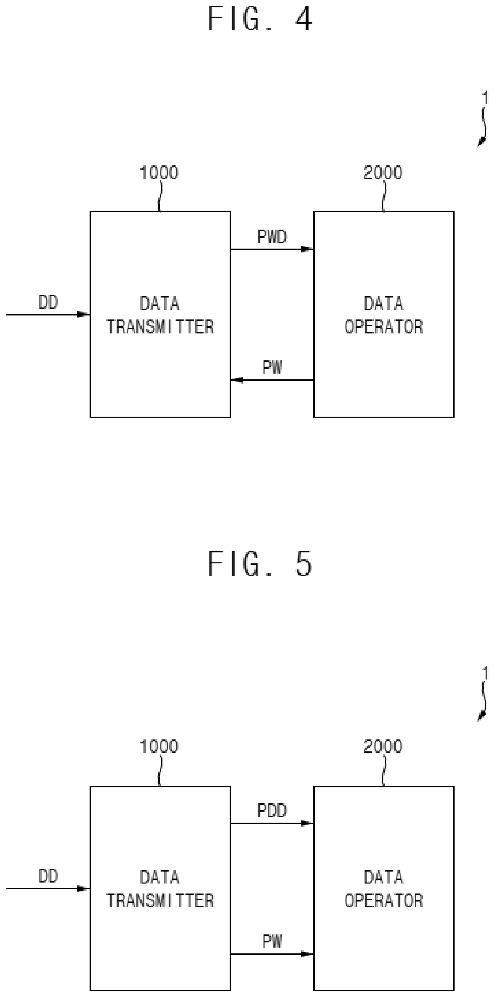

FIG. 3 is a flowchart illustrating a method for encrypting an oral data DD of FIG. 1. FIG. 4 is a block diagram illustrating an example of an operation of a prosthesis generating system 1 which performs the method for encrypting an oral data DD of FIG. 3. FIG. 5 is a block diagram illustrating an example of an operation of a prosthesis generating system 1 which performs the method for encrypting an oral data DD of FIG. 3.

Referring to FIG. 1 to FIG. 5, a method of encrypting oral data DD (S200) may include generating an encryption key PW of the oral data DD (5210), encrypting the oral data DD based on the encryption key PW (S220) and determining whether encrypted oral data PDD and the encryption key PW correspond (S230).

The data transmitter 1000 may output an encryption key operation request PWR to the data operator 2000. The data operator 2000 may output the encryption key PW to the data transmitter 1000 in response to the encryption key operation request PWR. The encryption key PW may correspond to the oral data DD received from the data transmitter 1000. The data transmitter 1000 may receive the encryption key PW. The data transmitter 1000 may encrypt the oral data DD based on the encryption key PW. Meta data of the encrypted oral data PDD may include the encryption key PW.

The data transmitter 1000 may output encrypted oral data PDD and the encryption key PW to the data operator 2000. The data operator 2000 may determine whether the encrypted oral data PDD and the encryption key PW correspond. When the encrypted oral data PDD and the encryption key PW correspond, the data operator 2000 may decrypt the encrypted oral data PDD. When the encrypted oral data PDD is decrypted, the data operator 2000 may generate prosthesis data CD based on the decrypted oral data. When the encrypted oral data PDD and the encryption key PW do not correspond, the data operator 2000 may not generate prosthesis data CD.

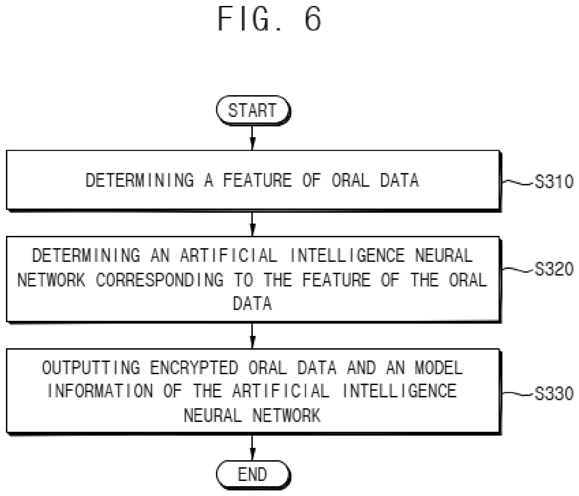

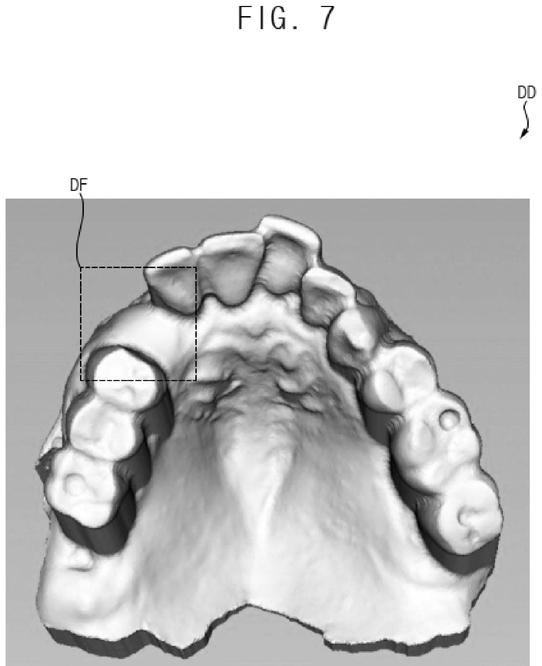

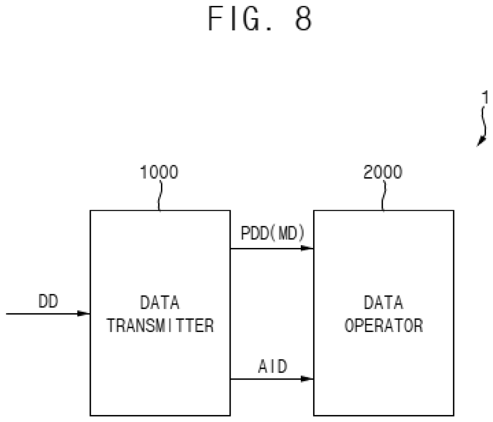

FIG. 6 is a flowchart illustrating a method for selecting an artificial intelligence neural network corresponding to the oral data DD of FIG. 1. FIG. 7 is a diagram illustrating a feature DF of the oral data DD of FIG. 6. FIG. 8 is a block diagram illustrating an example of an operation of a prosthesis generating system 1 which performs the method for selecting an artificial intelligence neural network corresponding to the oral data DD of FIG. 6.

Referring to FIG. 1 to FIG. 8, a method of selecting an artificial intelligence neural network corresponding to the oral data DD (S300) may include determining a feature DF of the oral data DD (S310), determining the artificial intelligence neural network corresponding to the feature DF of the oral data DD (S320) and outputting the encrypted oral data PDD and the model information AID of the artificial intelligence neural network (S330).

The data transmitter 1000 may determine the feature DF of the oral data DD. For example, the feature DF may refer to a tooth number of the oral data DD. For example, the feature DF may refer to the tooth number requiring the generation of a prosthesis. The data transmitter 1000 may determine an artificial intelligence neural network corresponding to the feature DF. In an embodiment, the data transmitter 1000 may determine the artificial intelligence neural network corresponding to the feature DF using the artificial intelligence neural network. The data transmitter 1000 may output the model information AID corresponding to the determined artificial intelligence neural network.

The data transmitter 1000 may output the encrypted oral data PDD. The encrypted oral data PDD may include an information of the oral data DD and meta data MD.

The meta data MD may include a tooth number, a tooth count, etc. of the oral data DD. For example, the tooth number may refer to a number of the tooth corresponding to the feature DF. For example, when the feature DF corresponds to the left maxillary central incisor, the tooth number may be 21. For example, the tooth count may refer to a count of tooth corresponding to the feature DF. For example, when the feature DF corresponds to the left maxillary central incisor and the left maxillary lateral incisor, the tooth count may be 2. However, the present inventive concept is not limited to types of features of the oral data DD included in the meta data MD. The meta data MD may further include prosthesis type data. For example, the prosthesis type data may include information of a crown, a laminate, an implant, etc.

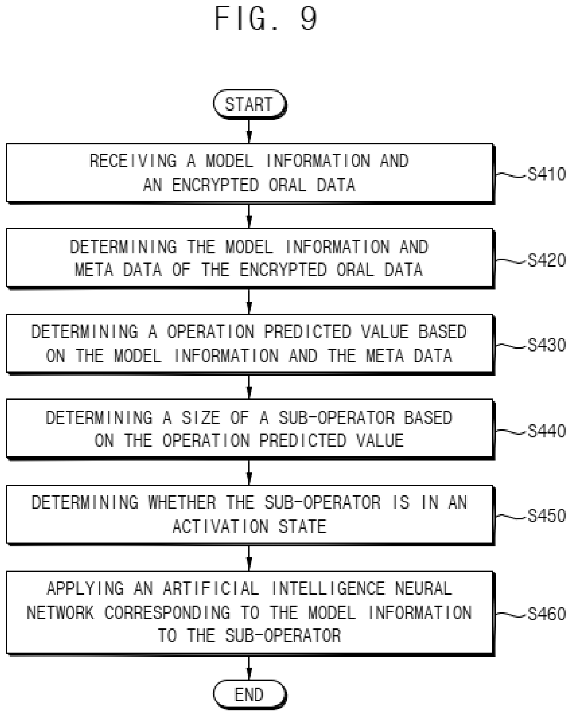

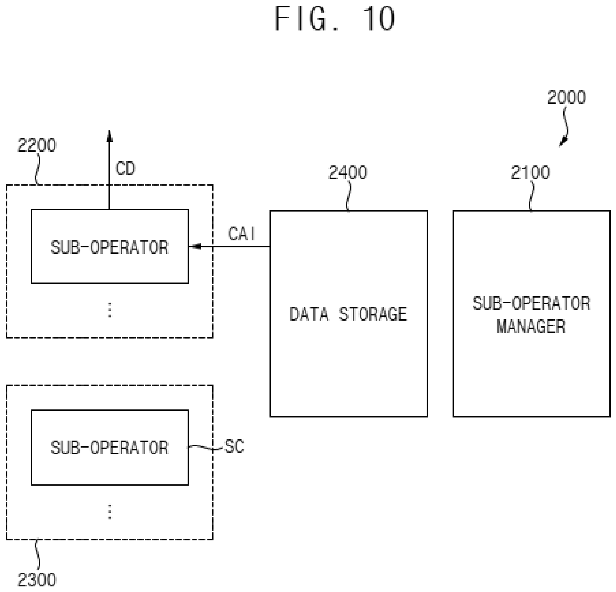

FIG. 9 is a flowchart illustrating a method for determining a sub-operator in which a selected artificial intelligence neural network is operated. FIG. 10 is a block diagram illustrating an example of an operation of a data operator 2000 included in the prosthesis generating system 1 of FIG. 1 which performs the method for determining a sub-operator in which a selected artificial intelligence neural network is operated.

Referring to FIG. 1 to FIG. 10, a method of determining a sub-operator SC in which a selected artificial intelligence neural network is operated (S400) may include receiving the model information AID and the encrypted oral data PDD (S410), determining the model information AID and the meta data MD of the encrypted oral data PDD (S420), determining an operation predicted value based on the model information AID and the meta data MD (S430), determining a size of the sub-operator SC based on the operation predicted value (S440), determining whether the sub-operator SC is in an activation state (S450) and applying an artificial intelligence neural network CAI corresponding to the model information AID to the sub-operator SC (S460).

The data operator 2000 may include a sub-operator manager 2100, an activation sub-operator block 2200, a waiting sub-operator block 2300 and a data storage 2400. Each of the activation sub-operator block 2200 and the waiting sub-operator block 2300 may include a sub-operator SC.

The sub-operator manager 2100 may control a state and a size of the sub-operator SC. The activation sub-operator block 2200 may include sub-operator SC which is in an activation state. The waiting sub-operator block 2300 may include sub-operator SC which is in a waiting state. The sub-operator SC may generate the prosthesis data CD corresponding to the oral data DD using the artificial intelligence neural network CAI corresponding to model information AID. The data storage 2400 may store a plurality of artificial intelligence neural networks. The data storage 2400 may apply the artificial intelligence neural network CAI corresponding to the model information AID to the sub-operator SC.

The sub-operator manager 2100 may control the state of the sub-operator SC. When the sub-operator SC is in an activation state, the sub-operator SC may generate the prosthesis data CD based on the artificial intelligence neural network CAI corresponding to the model information AID. When the sub-operator SC is in a waiting state, the sub-operator SC may wait in a state in which the artificial intelligence neural network CAI corresponding to the model information AID is stored. When the sub-operator SC is in an inactivation state, sub-operator SC which is in an inactivation state may be deleted. For example, when the sub-operator SC waits in the waiting state during a reference time, the sub-operator SC may be changed to an inactivation state. Accordingly, when the sub-operator SC waits in the waiting state during the reference time, the sub-operator SC may be deleted.

When the sub-operator SC storing the artificial intelligence neural network CAI corresponding to the model information AID is in the waiting state, the sub-operator manager 2100 may change a state of the sub-operation unit SC storing the artificial intelligence neural network CAI corresponding to the model information ID to the activation state. When the sub-operator SC storing the artificial intelligence neural network CAI corresponding to the model information AID is in the inactivation state (i.e., when the sub-operator SC storing the artificial intelligence neural network CAI corresponding to the model information AID is deleted), the sub-operator manager 2100 may generate the sub-operator SC. The data storage 2400 may apply the artificial intelligence neural network CAI corresponding to the model information AID to the generated sub-operator SC.

The data storage 2400 may store a plurality of artificial intelligence neural networks. Each of the artificial intelligence neural networks may be trained to generate the prosthetic data CD corresponding to the feature DF. For example, a first artificial intelligence neural network included in the artificial intelligence neural networks may be trained to generate the prosthetic data CD corresponding to a first tooth number. For example, the first artificial intelligence neural network may be trained to generate the prosthetic data CD corresponding to the first tooth number using information such as the first tooth number, surrounding teeth of the first tooth number, and antagonist tooth of the first tooth number. For example, a second artificial intelligence neural network included in the artificial intelligence neural networks may be trained to generate the prosthetic data CD corresponding to the first to third tooth numbers.

The sub-operator manager 2100 may control the size of the sub-operator SC based on the operation predicted value. For example, the size may refer to a size of a server space. The operation predicted value may be operated based on meta data MD and model information AID. For example, when a number of tooth count included in the meta data MD is greater than or equal to a reference tooth count, the size of the sub-operator SC may be increased. The reference tooth count may be set by a setter. For example, when the capacity of the artificial intelligence neural network corresponding to the model information AID is greater than or equal to a reference capacity, the size of the sub-operator SC may be increased. The reference capacity may be set by a setter. In an embodiment, when the size of the sub-operator SC corresponding to the tooth count and the model information AID is not operated, the sub-operator manager 2100 may generate a sub-operator SC having a reference size. The reference size may be set by a setter.

The sub-operator SC may generate the prosthesis data CD based on the oral data DD using the artificial intelligence neural network CAI corresponding to model information AID.

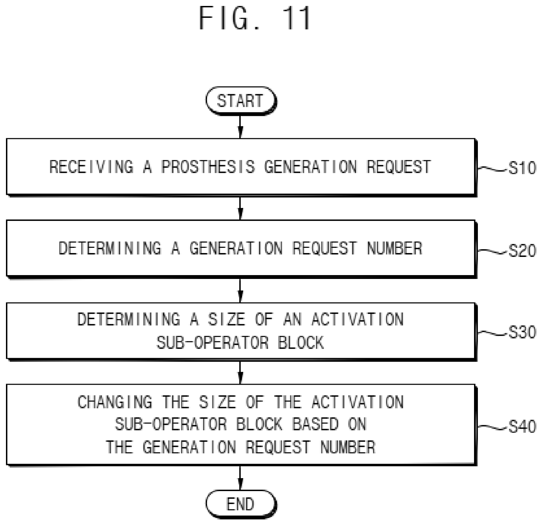

FIG. 11 is a flowchart illustrating an example of an operation of a data operator 2000 of FIG. 10.

Referring to FIG. 1 to FIG. 11, the data operator 2000 may change the size of the activation sub-operator block 2200. A method of changing the activation sub-operator block 2200 may include receiving a prosthesis generation request (S10), determining a generation request number (S20), determining the size of the activation sub-operator block 2200 (S30) and changing the size of the activation sub-operator block 2200 based on the generation request number (S40).

The generation request number may correspond to a number of the prosthesis generation request. For example, when the number of users requesting the prosthesis data CD increases, the number of generation requests may increase. For example, when the number of the prosthesis data CD requested by users increases, the generation request number may be increased.

The sub-operator manager 2100 may change the size of the activation sub-operator block 2200 based on the number of generation requests. For example, when the generation request number is increased, the sub-operator manager 2100 may increase the size of the activation sub-operator block 2200. For example, when the generation request number is greater than or equal to a reference generation request number, the sub-operator manager 2100 may increase the size of the activation sub-operator block 2200. For example, the reference generation request number may be set by a setter.

For example, when the generation request number is increased, the sub-operator manager 2100 may increase the number of sub-operator manager 2100 which is in an activation state. For example, when the generation request number is greater than or equal to the reference generation request number, the sub-operator manager 2100 may increase the number of sub-operator manager 2100 which is in an activation state.

In the present embodiment, the prosthesis data CD may be generated using a selected artificial intelligence neural network. Accordingly, the prosthesis data CD suitable for user's oral data DD may be generated. Additionally, a manufacturing speed of prosthesis may be improved.

Additionally, the size of the data operator 2000 may be changed based on the model information AID of the artificial intelligence neural network and the meta data MD of the oral data DD. Accordingly, an operating speed of the prosthesis data CD may be improved.

Additionally, the prosthetic data CD may be generated by the data operator 2000. Additionally, the data operator 2000 may be a cloud server environment. Accordingly, the operating speed of the prosthetic data CD may be further improved.

In an embodiment, the prosthesis generating method using three dimensional scan data and computer readable medium having program for performing the method as described in the above embodiments is recorded may be provided. The above-described method may be implemented as a program executable on a computer, and may be realized on a general-purpose digital computer that operates the program using a computer-readable medium. In addition, the structure of the data used in the above-described method may be recorded on the computer-readable medium by various means. The computer-readable medium may include program instructions, data files, data structures, and the like, either alone or in combination. The program instructions recorded on the medium may be those specifically designed and configured for the present inventive concept, or may be program instructions that are publicly known and usable by those skilled in the field of computer software. Examples of the computer-readable recording medium include magnetic media such as hard disks, floppy disks, and magnetic tapes, optical recording media such as CD-ROMs and DVDs, magneto-optical media such as floptical disks, and hardware devices such as ROM, RAM, and flash memory, which are specifically configured to store and execute program instructions. Examples of the program instructions include not only machine language code generated by compilers, but also high-level language code that may be executed by computers using interpreters and the like. The above-mentioned hardware devices may be configured to operate as one or more software modules for performing operations of the present inventive concept.

Additionally, the above-described a prosthesis generating method using three dimensional scan data may also be implemented in the form of a computer program or application executed by a computer and stored on a recording medium.

The present inventive concept relates to the prosthesis generating method using three dimensional scan data and computer readable medium having program for performing the method. A manufacturing speed of a prosthesis may be improved by using the prosthesis generating method using three dimensional scan data.

While the above has been described with reference to exemplary embodiments of the present inventive concept, it will be understood by those skilled in the art that the present inventive concept may be variously modified and changed without departing from the spirit and scope of the present inventive concept as defined in the following claims.

## Claims

1. A prosthesis generating method using three dimensional scan data:
receiving encryption oral data;
receiving a model information of an artificial intelligence neural network;
determining meta data of the encryption oral data;
determining an operation predicted value based on the model information and the meta data; and
determining a size of a sub-operator based on the operation predicted value,
wherein the size of the sub-operator is controlled based on the operation predicted value.

2. The method of claim 1, wherein the encryption oral data is generated based on oral data, and
wherein the artificial intelligence neural network is selected based on a feature of the oral data.

3. The method of claim 2, wherein when the sub-operator is in an inactivation state, the sub-operator is generated based on the size,
wherein the generated sub-operator receives the artificial intelligence neural network corresponding to the model information, and
wherein the generated sub-operator generates prosthesis data based on the received artificial intelligence neural network and the encryption oral data.

4. The method of claim 2, wherein when the sub-operator is in a waiting state, the sub-operator receives the artificial intelligence neural network corresponding to the model information, and
wherein the generated sub-operator generates prosthesis data based on the received artificial intelligence neural network and the encryption oral data.

5. The method of claim 4, wherein when the sub-operator generates the prosthesis data, a state of the sub-operator is changed to the waiting state.

6. The method of claim 1, wherein the meta data include an information of a tooth number and a tooth count of oral data corresponding to the encryption oral data, and
wherein when the tooth count is greater than a reference tooth count, the size of the sub-operator is increased.

7. The method of claim 6, wherein the meta data further includes prosthesis type data.

8. The method of claim 6, further comprising:
receiving a generation request number,
wherein when the generation request number is greater than a reference generation request number, a size of an activation sub-operation block which includes the sub-operator which is in an activation state is increased.

9. The method of claim 1, wherein the sub-operator receives the artificial intelligence neural network from a data storage,
wherein the data storage includes a plurality of artificial intelligence neural networks, and
wherein the data storage applies the artificial intelligence neural network corresponding to the model information to the sub-operator.

10. The method of claim 9, wherein the sub-operator generates prosthesis data based on the received artificial intelligence neural network and the encryption oral data.

11. The method of claim 1, further comprising:
receiving a generation request number,
wherein when the generation request number is greater than a reference generation request number, a size of an activation sub-operation block which includes the sub-operator which is in an activation state is increased.

12. The method of claim 1, further comprising:
receiving a generation request number,
wherein when the generation request number is greater than a reference generation request number, a number of the sub-operator which is in an activation state is increased.

13. The method of claim 1, wherein the model information is determined based on oral data corresponding to the encryption oral data, and
wherein the model information is determined based on a tooth number of the oral data and a prosthesis type.

14. The method of claim 1, wherein a method of generating the encryption oral data includes:
generating an encryption key of oral data; and
encrypting the oral data based on the encryption key, and
wherein when the encryption key and the encryption oral data are same, the encryption oral data is received.

15. A non-transitory computer-readable storage medium having stored thereon program instructions, the program instructions executable by at least one hardware processor to:
receive encryption oral data;
receive a model information of an artificial intelligence neural network;
determine meta data of the encryption oral data;
determine an operation predicted value based on the model information and the meta data; and
determine a size of a sub-operator based on the operation predicted value,
wherein the size of the sub-operator is controlled based on the operation predicted value.
